# EUROPEAN PATENT APPLICATION

(11) **EP 3 950 948 A1**
(43) Date of publication of application: **09.02.2022**
(21) Application number: 20190134.5
(22) Date of filing: 07.08.2020
(51) Int. Cl.: C12N 15/869, A61K 35/763, A61K 48/00

(54) **PLATFORM VECTOR FOR MODULAR AND SIMPLIFIED INSERTION OF TRANSGENES INTO ALPHAHERPESVIRINAE**

(71) Applicant: Fraunhofer-Gesellschaft zur Förderung der angewandten Forschung e.V., 80686 München (DE)
(72) Inventor: Bailer, Susanne, 70569 Stuttgart (DE); Funk, Christina, 70569 Stuttgart (DE)
(74) Representative: Wohlfahrt, Jan Günther

(57) **Abstract**

The present invention refers to a vector system, usable as a platform vector and suitable for the production of transgenic viruses of the subfamily *Alphaherpesvirinae.* Such transgenic viruses can be used as vaccine or as oncolytic virus or in gene therapy. The platform vector of the present invention is a vector system allowing a simplified search for and generation and production of viruses with a modified and increased functionality. The present invention refers also to the use of the platform vector as a vector system for the generation and the production of transgenic viruses, methods for the production of a transgenic virus, using the vector system of the present invention and viruses obtained by such methods.

## Description

The present invention refers to a vector system, usable as a platform vector and suitable for the production of transgenic viruses of the subfamily *Alphaherpesvirinae.* Such transgenic viruses can be used as vaccine or as oncolytic virus or in gene therapy. The platform vector of the present invention is a vector system allowing a simplified search for and generation and production of viruses with a modified and increased functionality. The present invention refers also to the use of the platform vector as a vector system for the generation and the production of transgenic viruses, methods for the production of a transgenic virus, using the vector system of the present invention and viruses obtained by such methods.

The *Alphaherpesvirinae* is a subfamily of viruses in the family *Herpesviridae,* primarily distinguished by reproducing more quickly than other subfamilies, by persistence in neurons and by a broad host range in mammals. *Alphaherpesvirinae* contain highly relevant viruses of animal and human virology. There are currently 42 species in the subfamily. Also, herpes simplex virus 1 (HSV-1) and herpes simplex virus 2 (HSV-2) and the closely related human alpha herpes virus 3 (HHV-3) belong to this subfamily.

Many variants of herpes simplex viruses have been considered for viral therapy of cancer, i.e. the use as oncolytic herpes virus. Such herpes viruses shall infect and kill cancer cells. As the infected cells are destroyed by lysis, they release new infectious virus particles or variants to help destroy the remaining tumour (S. Varghese and S.D. Rabkin, Cancer Gene Therapy (2002) 9, 967-978). The use of herpes virus vectors in gene therapy (V. Hukkanen, The Open Virology Journal (2010) 4, 94-95) and as vaccine vectors (P. Marconi et al., Human Vaccines (2008) 4(2), 91-105) is known too.

Bacterial artificial chromosomes (BAC) are DNA-constructs based on a functional fertility plasmid, used for transformation of and cloning in bacteria, usually *E.coli.* BACs can be used to clone large genomic sequences so that the bacterial artificial chromosomes can have usual insert sizes of 150 to 350 kbp. US 2012/0003742 A1 and M. Tanaka et al, J. Virol (2003) 77(2), 1382-1391, describe the cloning of herpes simplex virus type 1 (HSV-1) genomes as bacterial artificial chromosomes.

The according BACs of the state of the art are no suitable tools for the search for and generation of new and improved transgenic viruses to be produced in cells, e.g. mammalian cells afterwards.

It is therefore an object of the present invention to provide a tool, especially a vector, which allows for a simplified method to search for new suitable and especially improved transgenic viruses of the subfamily *Alphaherpesvirinae* and to generate these viruses for testing and/or to produce these viruses, especially such viruses which can be used as vaccines or as oncolytic viruses or in gene therapy.

It is further an object of the present invention to provide new transgenic viruses of the subfamily *Alphaherpesvirinae* and tools to develop and produce them, wherein the transgenic viruses have an improved functionality.

The problem underlying the present invention is solved by the provision of a vector system according to claim 1.

The present invention refers accordingly to a vector system comprising a bacterial artificial chromosome (BAC) construct, comprising a viral part and a non-viral part, wherein the viral part is derived of a virus of the subfamily *Alphaherpesvirinae*, wherein the non-viral part comprises DNA with sequences of a bacterial plasmid, wherein the viral part comprises at least two recombination sites.

The at least two recombination sites of the viral part are suitable or are used for functionalization, and especially for functionalization by the insertion of transgene expression cassettes.

In a preferred embodiment the viral part comprises two recombination sites. In a preferred embodiment the viral part has exactly two recombination sites for insertion of transgene expression cassettes comprising one or more genes (transgenes), i.e. open reading frames, and respective elements to allow for selection and to control their expression.

The at least two recombination sites according to the present invention allow advantageously a modular and simplified insertion of at least two transgene expression cassettes. The possibility to introduce not only one but at least two transgene expression cassettes allows the search for and the generation and the testing and production of new and more suitable transgenic viruses with new and combinable functions. Two transgene expression cassettes can be combined in a modular way to generate new transgenic viruses with a simplified method. The present invention allows for example the insertion of a transgene expression cassette into the first recombination site of the viral part and testing the interaction of the at least one product of this transgene expression cassette with different further products of transgene expression cassettes which can be inserted into the second recombination site of the viral part. It is advantageously possible to combine the functions and effects of two different transgene expression cassettes to improve the virus. Therefore, the vector system according to the present invention can be used advantageously as a platform vector for the generation and production of new transgenic viruses of the subfamily *Alphaherpesvirinae*, preferably transgenic herpes simplex virus 1 (HSV-1) or herpes simplex virus 2 (HSV-2). The vector system according to the present invention allows the insertion of at least two transgene expression cassettes, e.g. selected from transgene expression cassettes each encoding one or more proteins or peptides with a medical function for example selected from the group consisting of target control proteins, immunomodulating agents like immune-checkpoint inhibitors or cytokines, anti-cancer peptides (ACP) or proteins, pro-apoptotic proteins, extracellular matrix degrading proteins, proteins for tumor-antigen-presentation and pathogen antigens. Due to the combination of two or more of these transgene expression cassettes new and functionally improved transgenic viruses can be obtained.

The person skilled in the art knows suitable recombination sites like FRT and variants thereof like mFRT (mutated FRT as described e.g. in T. Schlake and J. Bode; Biochemistry. 1994;33(43):12746-12751), or like rox and loxP. In a preferred embodiment the at least two recombination sites are sites for a recombination with a site-specific recombinase. In a preferred embodiment the at least two recombination sites of the viral part are sites for a recombination with a Flp recombinase, with a Dre recombinase or with a Cre recombinase.

In a preferred embodiment the at least two recombination sites of the viral part are different recombination sites, e.g. the first recombination site is an FRT recombination site and the second recombination site is an mFRT recombination site. This has the advantage, that a specific insertion of a transgene or transgene cassettes into either of the recombination sites is possible.

In a preferred embodiment the at least two recombination sites of the viral part are an FRT or an mFRT or a rox or a loxP site. In a preferred embodiment one of the at least two recombination sites is an FRT or an mFRT or a rox or a loxP site. In a preferred embodiment one of the at least two recombination sites of the viral part is an FRT site. In a preferred embodiment one of the at least two recombination sites of the viral part is an mFRT site. In a preferred embodiment one of the at least two recombination sites of the viral part is an FRT site and the other is an mFRT site. In a preferred embodiment one of the at least two recombination sites can also be a loxP site or a rox site.

In a preferred embodiment the viral part is derived of an *Alphaherpesvirinae.* In a preferred embodiment the viral part is derived of herpes simplex virus 1 (HSV-1) or herpes simplex virus 2 (HSV-2) or human alphaherpesvirus 3 (HHV3).

In a preferred embodiment the viral part is derived of a Herpes simplex virus. In a preferred embodiment the viral part is derived of herpes simplex virus 1 (HSV-1) or herpes simplex virus 2 (HSV-2). In a preferred embodiment the viral part is derived of a herpes simplex virus 1 (HSV-1). In a preferred embodiment the viral part is derived of herpes simplex virus 2 (HSV-2). In an alternative embodiment the viral part is derived of human alphaherpesvirus 3 (HHV3).

In a preferred embodiment the viral part encodes an *Alphaherpesvirinae.* In a preferred embodiment the viral part encodes a Herpes simplex virus. In a preferred embodiment the viral part encodes herpes simplex virus 1 (HSV-1) or herpes simplex virus 2 (HSV-2) or human alphaherpesvirus 3 (HHV3).

In a preferred embodiment the viral part encodes herpes simplex virus 1 (HSV-1) or herpes simplex virus 2 (HSV-2). In a preferred embodiment the viral part encodes herpes simplex virus 1 (HSV-1). In a preferred embodiment the viral part encodes herpes simplex virus 2 (HSV-2). In a preferred embodiment the viral part encodes human alphaherpesvirus 3 (HHV3).

*Alphaherpesvirinae* are on one side relevant pathogens, for which improved vaccines are needed. *Alphaherpesvirinae* are on the other side well known and fully sequenced and therefor suitable tools for the use as oncolytic viruses, vaccine vectors or in gene therapy.

According to the present invention the vector system comprises a non-viral part and a viral part. The viral part refers to DNA-sequences with the origin of a virus of the subfamily *Alphaherpesvirinae.* According to the present invention the viral part comprises at least two recombination sites. These recombination sites do not have to be of viral origin. These at least two recombination sites are inserted into the viral-part and therefore lie in the viral part. As outlined above, these at least two recombination sites are preferably not of viral origin, but are recombination sites like FRT, mFRT, loxP or rox.

The non-viral part refers to other DNA-sequences, especially bacterial sequences. The non-viral part can also comprise eukaryotic sequences. The non-viral part can also comprise viral sequences excluding coding sequences of viruses of the subfamily *Alphaherpesvirinae.* The non-viral part can comprise therefore especially also sequences of bacteriophages, e.g. bacteriophage sequences used in bacterial plasmids.

In a preferred embodiment the non-viral part comprises only eukaryotic and/or bacterial sequences or only eukaryotic and/or bacterial sequences in combination with bacteriophage sequences.

According to the present invention the non-viral part comprises DNA with sequences of a bacterial plasmid. These sequences can preferably be the BAC cassette, i.e. the sequences building the BAC core of the vector system of the present invention, e.g. sequences of a plasmid like the plasmid pBeloBAC11. The non-viral part can comprise further DNA-sequences. The further DNA-sequences of the non-viral part can be directly attached to the sequences building the BAC core of the vector system or can be separated from the sequences building the BAC core of the vector system, e.g. by DNA-sequences of the viral part. In a preferred embodiment the further DNA-sequences of the non-viral part are directly attached to the sequences building the BAC core of the vector system. In a more preferred embodiment the non-viral part is separated from the viral part by two recombination sites, more preferably by two recombination sites of the same kind, most preferably by two loxP recombination sites. These two recombination sites, e.g. loxP recombination sites of the non-viral part are distinct from the two recombination sites of the viral part used to search for and to generate and produce functionalized vectors and viruses.

According to the present invention the vector system has a viral part and a non-viral part, wherein the viral part comprises at least two recombination sites. These at least two recombination sites can be used to introduce transgene expression cassettes. The transgene expression cassettes can be of any kind of origin and are inserted into the recombination sites of the viral part but are entities distinct from the viral and non-viral part of the vector system.

The present invention refers to the vector system with or without transgene expression cassettes introduced into the recombination sites of the viral part.

In a preferred embodiment the DNA-sequences of the transgene expression cassettes are separated from the non-viral part and especially from the sequences building the BAC core of the vector system by DNA-sequences of the viral part after insertion into the at least two recombination sites of the viral part.

In a preferred embodiment the DNA-sequences of the at least two transgene expression cassettes are separated from each other by DNA-sequences of the viral part after insertion into the at least two recombination sites of the viral part.

In a preferred embodiment the DNA with sequences of a bacterial plasmid of the non-viral part is inserted in between the sequences of a first viral gene and a second viral gene.

In a preferred embodiment the non-viral part is inserted in between the sequences of a first viral gene and a second viral gene.

In a preferred embodiment the first recombination site of the viral part is inserted in between the sequences of two viral genes. In a preferred embodiment the first recombination site of the viral part is inserted in between the sequences of two viral genes and the second recombination site of the viral part is inserted in between the sequences of two further viral genes.

In a preferred embodiment the non-viral part is inserted in between the sequences of a first viral gene and a second viral gene and the first recombination site of the viral part is inserted in between the sequences of a third viral gene and a fourth viral gene and the second recombination site of the viral part is inserted in between the sequences of a fifth viral gene and a sixth viral gene.

In a preferred embodiment the DNA with sequences of a bacterial plasmid of the non-viral part is inserted in between the sequences of a first viral gene and a second viral gene and the first recombination site of the viral part is inserted in between the sequences of a third viral gene and a fourth viral gene and the second recombination site of the viral part is inserted in between the sequences of a fifth viral gene and a sixth viral gene.

In a preferred embodiment the viral part comprises at least 50%, more preferably at least 65%, most preferably at least 80% of the encoding sequences of a virus of the subfamily *Alphaherpesvirinae*, preferably herpes simplex virus 1 (HSV-1) or herpes simplex virus 2 (HSV-2) or human alphaherpesvirus 3 (HHV3).

In a preferred embodiment the viral part comprises at least 50%, more preferably at least 65%, most preferably at least 80% of the encoding sequences of a virus of the subfamily *Alphaherpesvirinae*, preferably a Herpes simplex virus, most preferably herpes simplex virus 1 (HSV-1) or herpes simplex virus 2 (HSV-2).

In a preferred embodiment the viral part comprises at least 25 encoding sequences, more preferably 30 encoding sequences, even more preferably 40 encoding sequences out of the 56 encoding sequences UL1 to UL56.

In a preferred embodiment the viral part comprises at least 5 encoding sequences, more preferably 6 encoding sequences, even more preferably 9 encoding sequences out of the 12 encoding sequences US1 to US 12.

In a preferred embodiment the viral part comprises at least 25 encoding sequences, more preferably 30 encoding sequences, even more preferably 40 encoding sequences out of the 56 encoding sequences for the gene-products UL1 to UL56 and at least 5 encoding sequences, more preferably 6 encoding sequences, even more preferably 9 encoding sequences out of the 12 encoding sequences for the gene-products US1 to US 12. In a preferred embodiment the viral part comprises at least some of the encoding genes of the inverted repeats of the viral genome, e.g. the terminal repeats and/or the internal repeats.

The skilled person knows suitable and necessary open reading frames (ORF) of the *Alphaherpesvirinae*, especially herpes simplex virus 1 (HSV-1), herpes simplex virus 2 (HSV-2) or human alphaherpesvirus 3 (HHV3), which can be used for the viral part of the vector system.

In a preferred embodiment the viral part comprises all encoding sequences out of the 56 encoding sequences for the gene-products UL1 to UL56 and out of the 12 encoding sequences for the gene-products US1 to US 12.

The encoding sequences UL1 to UL56 and US1 to US12 and the gene products of Herpes Simplex Virus and respective homologs thereof in other *Alphaherpesvirinae* are known to the skilled person and described e.g. in D.J. McGeoch, et al., Journal of General Virology (1988). 69 (7): 1531-1574, in D.J. McGeoch, et al., Journal of Molecular Biology, (1985) 181(1): 1-13, in T Lenac Roviš, SM Bailer, et al., Journal of Virology (2013), 87 (12): 6943-54, in BG Klupp et al., Journal of Virology (2004);78(1):424-440 and in A. Dolan et al., Journal of Virology (1998), 72(3): 2010-2021.

In a preferred embodiment the non-viral part is inserted in between the sequences of two tail-to-tail oriented genes or open reading frames.

In a preferred embodiment the non-viral part is inserted in between the sequences of the UL3 gene and the UL4 gene.

The intergenic region of two tail-to-tail oriented genes like UL3 and UL4 is especially suitable since the tail-to-tail orientation of genes generates an intergenic region where insertion of a foreign gene like the non-viral part between two tail-to-tail oriented polyadenylation signals does not disrupt any viral genes or transcriptional units.

Further accordingly suitable insertion sites are between the sequences of the UL7 gene and the UL8 gene, between the sequences of the UL10 gene and the UL11 gene, between the sequences of the UL15 gene and the UL18 gene, between the sequences of the UL21 gene and the UL22 gene, between the sequences of the UL26 gene and the UL27 gene, between the sequences of the UL35 gene and the UL36 gene, between the sequences of the UL40 gene and the UL41 gene, between the sequences of the UL45 gene and the UL46 gene, between the sequences of the UL55 gene and the UL56 gene and between the sequences of the US9 gene and the US10 gene.

In a preferred embodiment at least one of the at least two recombination sites of the viral part is inserted in between the sequences of two tail-to-tail oriented genes. In a preferred embodiment both of the at least two recombination sites of the viral part are inserted in between the sequences of two tail-to-tail oriented genes. In a preferred embodiment all of the at least two recombination sites of the viral part are inserted in between the sequences of two tail-to-tail oriented genes.

In a preferred embodiment one of the at least two recombination sites of the viral part is inserted in between the sequences of the UL10 gene and the UL11 gene. In a preferred embodiment one of the at least two recombination sites of the viral part is inserted in between the sequences of the UL55 gene and the UL56 gene. In a preferred embodiment one of the at least two recombination sites of the viral part is inserted in between the sequences of the UL10 gene and the UL11 gene and the other of the at least two recombination sites of the viral part is inserted in between the sequences of the UL55 gene and the UL56 gene. The other intergenic region of two tail-to-tail oriented genes outlined above, namely between the sequences of the UL7 gene and the UL8 gene, between the sequences of the UL3 gene and the UL4 gene, between the sequences of the UL15 gene and the UL18 gene, between the sequences of the UL21 gene and the UL22 gene, between the sequences of the UL26 gene and the UL27 gene, between the sequences of the UL35 gene and the UL36 gene, between the sequences of the UL40 gene and the UL41 gene, between the sequences of the UL45 gene and the UL46 gene, and between the sequences of the US9 gene and the US 10 gene are also preferred.

In a preferred embodiment the at least two recombination sites of the viral part are inserted in distance, more preferably large distance. In a preferred embodiment between the two recombination sites of the viral part are located at least 5 genes, more preferably at least 20 genes, even more preferably at least 30 genes, even more preferably at least 40 genes. Preferably these genes between the at least two recombination sites of the viral part are UL genes.

A distance, more preferably large distance between the at least two recombination sites may have the advantage that steric hindrances after the insertion of more than one transgene expression cassette can be avoided.

In a preferred embodiment the non-viral part and the at least two recombination sites of the viral part are inserted in between the sequences of two tail-to-tail oriented genes, more preferably the non-viral part and the at least two recombination sites of the viral part are inserted in a different intergenic region selected from the group consisting of between the sequences of the UL3 gene and the UL4 gene, between the sequences of the UL7 gene and the UL8 gene, between the sequences of the UL10 gene and the UL11 gene, between the sequences of the UL15 gene and the UL18 gene, between the sequences of the UL21 gene and the UL22 gene, between the sequences of the UL26 gene and the UL27 gene, between the sequences of the UL35 gene and the UL36 gene, between the sequences of the UL40 gene and the UL41 gene, between the sequences of the UL45 gene and the UL46 gene, between the sequences of the UL55 and the UL56 gene and between the sequences of the US9 gene and the US 10 gene.

In a preferred embodiment at least one gene of the viral part is mutated. In a preferred embodiment at least one UL gene is mutated. In a preferred embodiment at least two genes of the viral part are mutated. In a preferred embodiment at least two UL genes are mutated. In a preferred embodiment the at least one mutated gene is a virulence gene. In a preferred embodiment at least two virulence genes are mutated. In a preferred embodiment two virulence genes are mutated. In a preferred embodiment UL37 is mutated. In a preferred embodiment UL39 is mutated. In a preferred embodiment at least UL37 and UL39 are mutated. In a preferred embodiment UL37 and UL39 are mutated. In a preferred embodiment the mutation is a point mutation or a deletion. In a preferred embodiment there are point mutations in the UL37 gene and a deletion in the UL39 gene.

The mutation in virulence genes like UL37 and/or UL39 has the advantage that the neurotoxicity of the virus can be avoided and the safety of the virus can be optimized.

In a preferred embodiment the virus where preferably at least one gene of the viral part is mutated is produced in a transcomplementing cell line expressing the respective viral gene, e.g. UL39. In a preferred embodiment the virus is produced in a transcomplementing cell line expressing UL39 gene under the control of a regulable promoter.

In a preferred embodiment the non-viral part comprises sequences of the plasmid pBeloBAC11.

In a preferred embodiment the non-viral part comprises an additional polyadenylation signal.

In a preferred embodiment the non-viral part comprises a selection cassette.

In a preferred embodiment the non-viral part can be removed after transfection in mammalian cells by recombination.

In a preferred embodiment the non-viral part, and more preferably the DNA with sequences of a bacterial plasmid, is framed by recombination sites, preferably a recombination site different from the two recombination sites inserted in the viral part. This has the advantage that the DNA with sequences of a bacterial plasmid, e.g. the sequences of the plasmid pBeloBAC11, can be removed when the transgenic viruses are produced in mammalian cells.

In a preferred embodiment the non-viral part, and more preferably the DNA with sequences of a bacterial plasmid, comprises a Cre-recombination cassette and is flanked by loxP sites. This has the advantage that the non-viral part in between the two loxP sites is removed when the transgenic viruses are produced in mammalian cells and only one loxP-recombination site remains.

In a preferred embodiment the non-viral part comprises sequences of the plasmid pBeloBAC11 and a Cre-recombination cassette flanked by loxP sites.

Accordingly, according to a preferred embodiment, the non-viral part comprises a first recombination site, e.g. loxP, at one end and a second recombination site of the same kind as the first recombination site at the other end. These recombination sites are distinct from the two recombination sites of the viral part used to search for and to generate and to produce functionalized vectors and viruses. In between the two recombination sites of the non-viral part is the DNA with sequences of a bacterial plasmid and preferably further sequences like a Cre-recombination cassette and/or a selection marker cassette. In a further preferred embodiment, there is an additional polyadenylation signal outside the two recombination sites of the non-viral part.

Accordingly, in a preferred embodiment, the vector system comprises two different recombination sites in the viral part and two further recombination sites at the ends of the non-viral part, wherein the two recombination sites of the non-viral part are of the same kind, but different from the two recombination sites of the viral part.

The present invention refers also to the use of the vector system according to the present invention for the generation and/or production of a transgenic virus. In a preferred embodiment the transgenic virus comprises two transgene expression cassettes.

According to the present invention a transgene expression cassette comprises one or more genes (transgenes), i.e. open reading frames, and the sequences to allow for selection and to control their expression. In a preferred embodiment the transgene expression cassette comprises at least one transgene. In a preferred embodiment the two transgene expression cassettes comprise each at least one transgene. In a preferred embodiment the two transgene expression cassettes consist each of one transgene. In a preferred embodiment one of the two transgene expression cassettes consist of one transgene. In a preferred embodiment one of the two transgene expression cassettes comprises at least two transgenes.

The present invention refers also to the vector system according to the present invention for the use for the generation and/or production of a transgenic virus. In a preferred embodiment the transgenic virus comprises two transgene expression cassettes.

The present invention refers also to a method for the production of a transgenic virus comprising the steps:
a) introducing a transgene expression cassette into one of the at least two recombination sites of the viral part of the vector system according to the present invention to obtain a vector encoding a transgenic virus;
b) transfecting the vector obtained in step a) into a mammalian cell;
c) cultivating the transfected mammalian cell;
d) isolating the virus produced in the mammalian cell.

In a preferred embodiment in step a) a first transgene expression cassette is introduced into the first of the at least two recombination sites and a second transgene expression cassette is introduced into the second of the at least two recombination sites of the viral part of the vector system.

The skilled person knows according methods for the introduction of transgenes and transgene expression cassettes, especially with the two recombination sites provided, and especially when two different recombination sites are provided. In a preferred embodiment step a) is performed in a prokaryotic system. Alternatively, a eukaryotic system, preferably a mammalian cell line can be used.

Preferably the mammalian cell of steps b) to d) is a mammalian cell line. Preferably the cell line is a Vero cell line.

The production of the transgenic virus according to steps b) to d) can either be performed in a non-complementing cell line if no essential genes of the virus were deleted or it can be performed in a trans-complementing cell line, especially if the transgenic virus shall be used as vaccine.

In a preferred embodiment the transgenes within the transgene expression cassettes encode a protein or a peptide or an RNA. In a preferred embodiment at least one of the transgenes encodes a protein or a peptide. In a preferred embodiment the transgene or at least one of the transgenes within the transgene expression cassettes encodes a protein or a peptide with a medical function. In a preferred embodiment the transgene or at least one of the transgenes within the transgene expression cassettes encodes a protein or a peptide selected from the group consisting of target control proteins, immunomodulating agents like immune-checkpoint inhibitors or cytokines, anti-cancer peptides (ACP) or proteins, pro-apoptotic proteins, extracellular matrix degrading proteins, proteins for the tumor-antigen-presentation and pathogen antigens.

In a preferred embodiment, the first transgene expression cassette and the second transgene expression cassette each encode at least one protein or at least one peptide or an RNA, preferably at least one protein or at least one peptide selected from the group consisting of target control proteins, immunomodulating agents like immune-checkpoint inhibitors or cytokines, anti-cancer peptides (ACP) or proteins, pro-apoptotic proteins, extracellular matrix degrading proteins, proteins for the tumor-antigen-presentation, pathogen antigens and combinations thereof.

The transgenes of the transgene expression cassettes can be under control of a constitutive promoter or a regulable promoter. A promoter can be a Herpes simplex virus derived promoter, an inducible (positively regulable) promoter, or a negatively regulable promoter. A negative regulation can be used advantageously for the production of oncolytic viruses and for the expression of cell damaging proteins.

The first transgene within the first transgene expression cassette can be under the control of a different promoter than the second transgene within the second transgene expression cassette. A transgene, which shall be or is introduced into one of the at least two recombination sites of the vector system can be a single open reading frame encoding a single protein, peptide or RNA. A transgene cassette, which shall be or is introduced into one of the at least two recombination sites of the viral part of the vector system can also comprise more than one open reading frames. Therefore, it is possible to produce with the at least two recombination sites more than two different proteins or peptides.

If a transgene expression cassette is introduced into one of the at least two recombination sites of the viral part and the transgene expression cassette comprises more than one transgene, i.e. more than one open reading frame, the different transgenes of the transgene expression cassette can be under the regulation of the same promoter or under the regulation of different promoters.

In a preferred embodiment the method according to the present invention is used for the production of a vaccine or an oncolytic virus or viral vector for gene therapy.

The present invention refers also to a virus obtained in the method according to the present invention.

In a preferred embodiment the virus is for the use as vaccine or as oncolytic virus or in a gene therapy.

In a preferred embodiment the virus is an oncolytic virus for treatment of non-small-cell lung carcinoma (NSCLC), preferably with two transgene expression cassettes, namely a target control protein for targeting to epidermal growth factor receptor (EGFR) expressing tumor cells and an immune checkpoint inhibitor against programmed cell death protein 1 (PD-1).

The present invention refers also to a vaccine or an oncolytic virus or a viral vector comprising a virus according to the present invention.

Further preferred embodiments are outlined in the dependent claims.

It should be noted that embodiments and features described in the context of one of the aspects of the present invention also apply to the other aspects of the invention. It should be noted that embodiments and features described in the context of the vector system according to the invention, may also be combined with embodiments and features described in the context of the methods and viruses of the invention and vice versa, unless expressly stated otherwise.

All patent and non-patent references cited in the present application, are hereby incorporated by reference in their entirety. Preferred embodiments of the present invention are described also in the following in the non-limiting examples and the figures.
- Figure 1: shows schematically a vector system according to the present invention and a virus produced from the vector system;
- Figure 2: shows the verification of stable insertion of two recombination sites into the HSV-1 genome;
- Figure 3: shows schematically a further vector system according to the present invention;
- Figure 4: shows the verification of the stable insertion of two transgene expression cassettes at the recombination sites, also called functionalisation;
- Figure 5: shows the detailed analysis of the functionalized virus platformNA-ICI-T.

### Examples

In the examples the advantage of the vector system according to the present invention is shown by insertion of a target control protein and an immune checkpoint inhibitor to obtain an oncolytic virus for treatment of non-small-cell lung carcinoma (NSCLC), more specifically a target control protein for targeting to epidermal growth factor receptor (EGFR) expressing tumor cells and an immune checkpoint inhibitor against programmed cell death protein 1 (PD-1). The immune checkpoint inhibitor corresponds to a single chain variable fragment (scFv) derived of an anti-PD-1 antibody. The target control protein consists of an scFv against EGFR N-terminally fused to HSV-1 glycoprotein H. As a result, a functionalized transgenic virus for a combined virus immune therapy was produced. The main advantage is the simplified, modular and stable insertion of two different transgene expression cassettes to functionalize the vector that allows different transgene combinations.

### Example 1

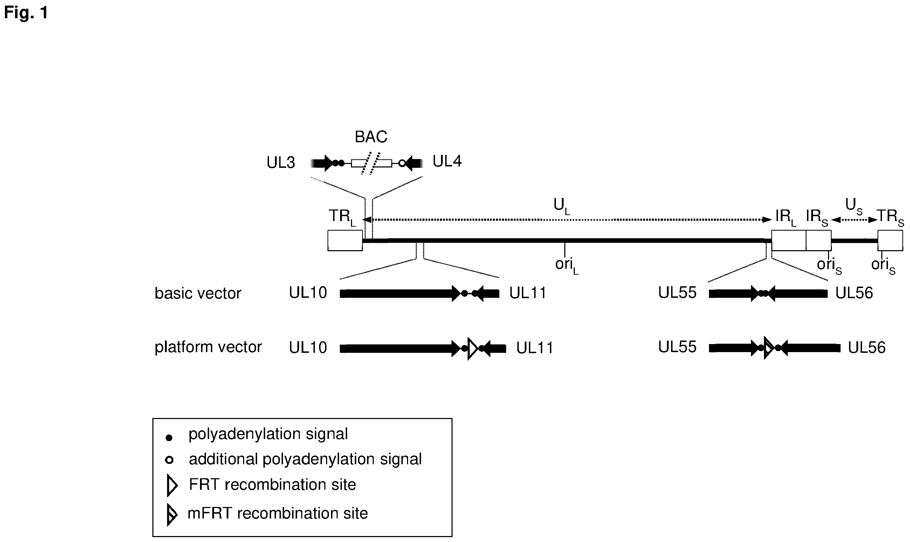

Figure 1 shows the schematic representation of the vector system according to the present invention before (basic vector) and after insertion of the recombination sites (platform vector). The genome of HSV-1 consisting of the regions UL (unique long), US (unique short) and the inverted repeats TR (terminal repeat, Terminal repeat long (TRL) and Terminal repeat short (TRS)) and IR (internal repeat, Inverted repeat long (IRL) and Inverted repeat short (IRS)) is depicted. Furthermore, the HSV-1 genome contains three origins of DNA replication of two types: one copy of oriL located at the center of the unique long (UL) region of the genome and two copies of oriS located in the repeats flanking the unique short (US) region of the genome. The non-viral part, here shown as BAC, including sequences of pBeloBAC11, a sequence encoding Cre recombinase and a Zeocin selection cassette, is flanked by loxP recombination sites and inserted between UL3 and UL4 together with an additional polyadenylation signal. The two recombination sites to generate the platform vector, FRT and mFRT, are inserted between UL10 and UL11 or UL55 and UL56, respectively. The core plasmid system with and for the recombination sites was kindly provided by Z. Ruzsics.

In Figure 1 following symbols are used:

| | |
|---|---|
| ● | polyadenylation signal |
| ∘ | additional polyadenylation signal |
| 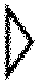 | FRT recombination site |
| 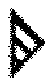 | mFRT recombination site |

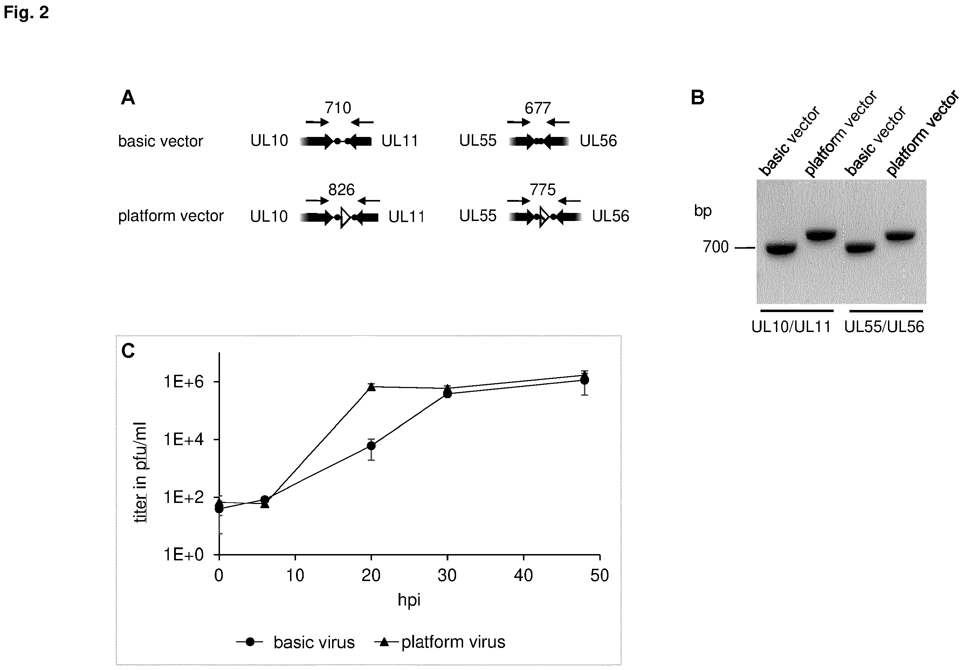

Figure 2 shows the verification of stable insertion of two recombination sites into the HSV-1 genome. A: Schematic depiction of the fragment sizes before (basic vector) and after insertion of the recombination sites (platform vector) starting from oligonucleotides (depicted as arrows) that bind in UL10 and UL11 or UL55 and UL56. Numbers between two oligonucleotides correspond to number of base pairs. B: Fragments of polymerase chain reactions (PCR) using oligonucleotides shown in A and the basic or platform vector as template. The fragments were separated in an agarose gel (2 %, Tris acetic acid EDTA buffer) and visualized by Midori Green Advance under UV light. C: Basic and platform virus were reconstituted by transfection of Vero cells with respective BAC DNA and further cultivation. Growth properties of basic and platform virus were determined by infection of Vero cells (MOI of 0,1) in triplicates, harvest of the cell culture supernatant at different hours post infection (hpi) and titration on Vero cells using plaque assay.

### Example 2

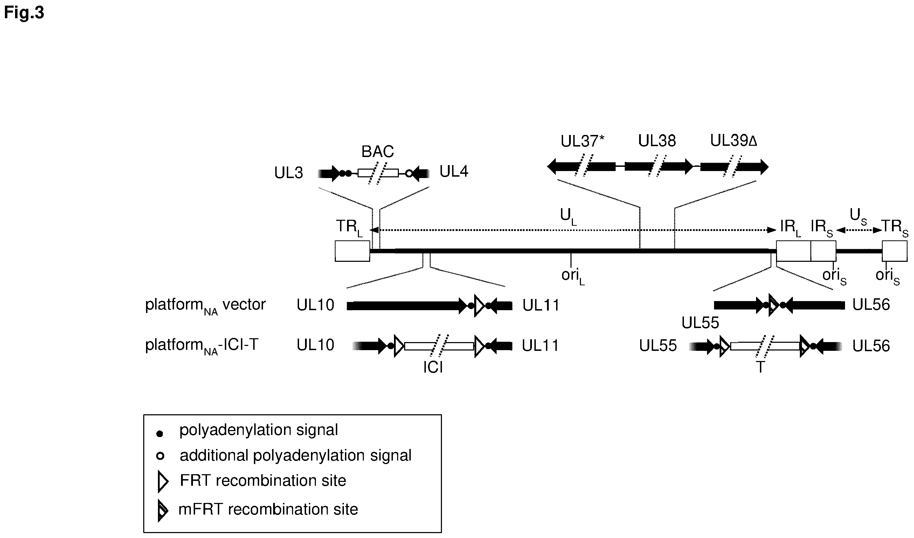

Figure 3 shows a schematic depiction of the vector system according to the present invention after functionalisation with two transgenes based on the schematic depiction of figure 1. The two virulence genes UL37 and UL39 contain point mutations and a partial deletion, respectively, to obtain an attenuated transgenic virus that is deficient in neuotoxicity (called platformNA). The functionalized vector platformNA-ICI-T is generated by Flp-mediated insertion of two transgenes at the recombination sites, FRT and mFRT. One transgene expression cassette depicted as ICI encodes an immune checkpoint inhibitor against programmed cell death protein 1 (PD-1), more specifically a single chain variable fragment (scFv) derived of an antibody against PD-1. The other transgene expression cassette depicted as T encodes a target control protein against the epidermal growth factor receptor (EGFR) expressed on tumor cells, more specifically the target control protein consists of an scFv against EGFR (kindly provided by R. Kontermann) N-terminally fused to HSV-1 glycoprotein H.

In Figure 3 following symbols are used:

| | |
|---|---|
| ● | polyadenylation signal |
| ∘ | additional polyadenylation signal |
| 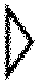 | FRT recombination site |
| 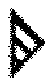 | mFRT recombination site |

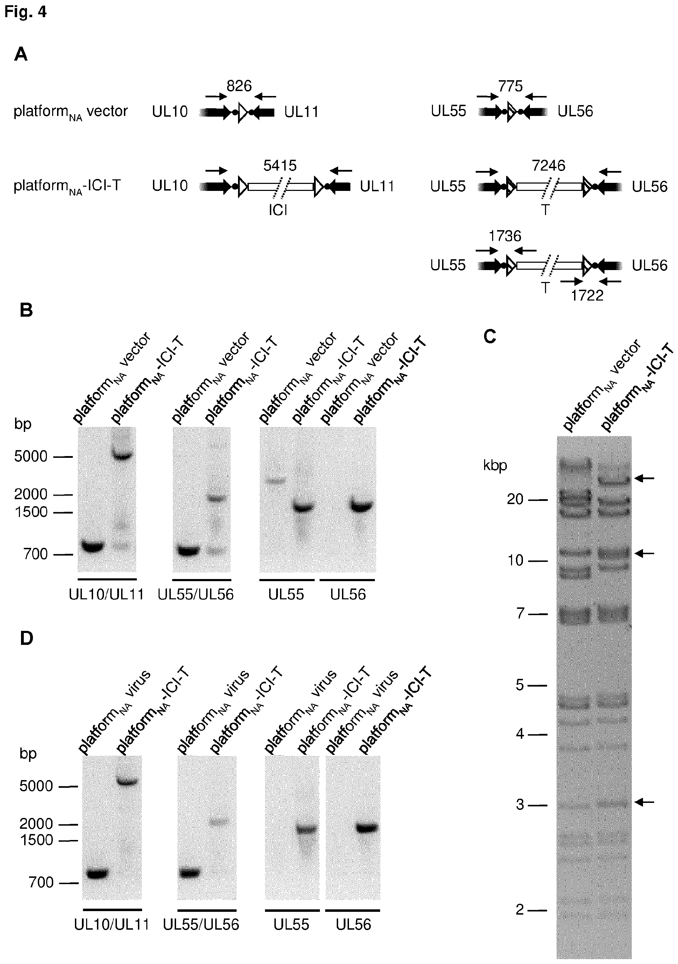

Figure 4 shows the verification of the stable insertion of two transgene expression cassettes at the recombination sites, also called functionalisation. A: Schematic depiction of the fragment sizes before (platform_{NA} vector) and after insertion of the transgene expression cassettes (platform_{NA}-ICI-T) starting from oligonucleotides (depicted as arrows) that bind in UL10 and UL11 or UL55 and UL56 or UL55/UL56 and the transgene expression cassettes comprising the target control protein. Numbers between two oligonucleotides correspond to length of fragments/products in base pairs produced by polymerase chain reactions (PCR) separated in B and D. B: Fragments of polymerase chain reactions (PCR) using oligonucleotides shown in A and the platform_{NA} or the platform_{NA}-ICI-T vector as template. The fragments were separated in an agarose gel (1,2 %, Tris acetic acid EDTA buffer) and visualized by Midori Green Advance under UV light. C: Isolated BAC DNA was digested with the restriction enzyme NotI, fragments were separated in an agarose gel (0,6 %, Tris boric acid EDTA buffer) and visualized by ethidium bromide under UV light. Expected differences regarding the restriction pattern between the platform_{NA} vector and the functionalized vector platform_{NA}-ICI-T are highlighted with arrows. D: PCR fragments as described in B but with platform_{NA} virus (passage 4) and platform_{NA}-ICI-T virus (passage 8) as template representing the stability of the transgene expression cassette insertion using the two recombination sites. Both viruses were reconstituted by transfection of Vero cells with the respective BAC DNA and further cultivation.

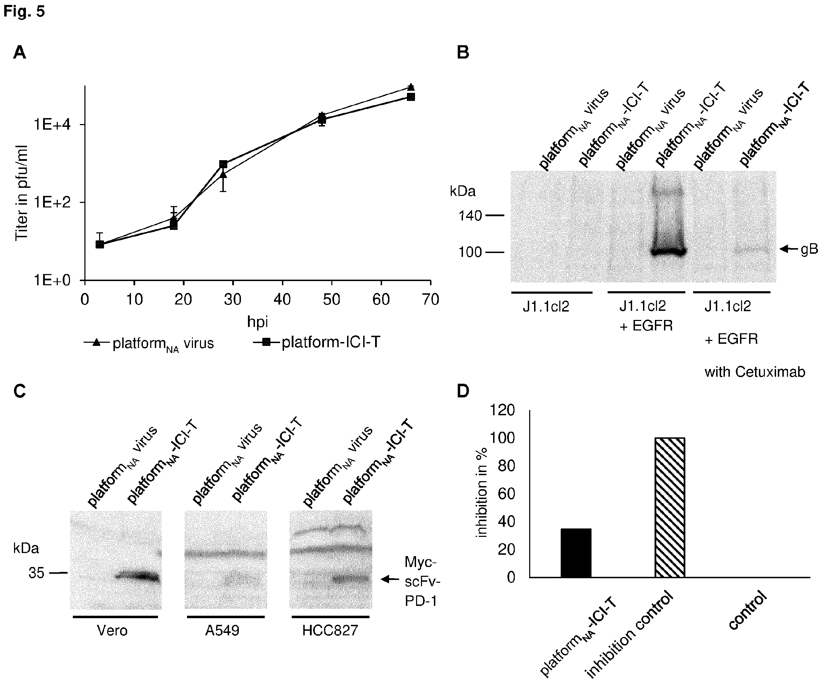

Figure 5 shows the detailed analysis of the functionalized virus platformNA-ICI-T including growth properties and expression as well as functional analysis of proteins encoded by inserted transgenes. A: Growth properties of platformNA virus compared to platformNA-ICI-T virus reconstituted as described in Figure 4D were determined by infection of Vero cells (MOI of 0,1) in triplicates, harvest of the cell culture supernatant at different hours post infection (hpi) and titration on Vero cells using plaque assay. B: Functional analysis of the targeting to EGFR expressing cells was investigated by infection (MOI of 5) of J1.1cl2 cells and J1.1cl2 cells expressing EGFR (suitable for the scFv used), either alone or in presence of Cetuximab (anti-EGFR antibody). 16 hours post infection cell lysates were harvested and analysed by SDS-PAGE followed by Western Blot to detect the HSV-1 glycoprotein B (gB) as an indicator for infection. J1.1cl2, kindly provided by G. Campadelli-Fiume, cannot be infected by HSV-1. C: Expression of the scFv against PD-1 was analysed in Vero cells as well as in the non-small-cell lung carcinoma (NSCLC) cell lines A549 und HCC827. 16 hours post infection (MOI of 0,5) cell lysates were harvested and analysed by SDS-PAGE and Western Blot to detect the Myc-labelled scFv. D: To test the functionality of the virus encoded scFv against PD-1 as an immune checkpoint inhibitor a commercially available PD-1:PD-L1 inhibitor screening assay was applied. Therefore Vero cells were infected (MOI of 0,5), 16 hours post infection cell lysates were harvested and tested in the PD-1:PD-L1 inhibitor screening assay. Pembrolizumab, an anti-PD-1 antibody, was used as inhibition control.

## Claims

1. A vector system comprising a bacterial artificial chromosome (BAC) construct, comprising a viral part and a non-viral part, wherein the viral part is derived of a virus of the subfamily *Alphaherpesvirinae*, wherein the non-viral part comprises DNA with sequences of a bacterial plasmid, **characterized in that** the viral part comprises at least two recombination sites.

2. The vector system according to claim 1, wherein the viral part is derived of a Herpes simplex virus.

3. The vector system according to the preceding claims, wherein the DNA with sequences of a bacterial plasmid is inserted in between the sequences of a first viral gene and a second viral gene and the first recombination site is inserted in between the sequences of a third viral gene and a fourth viral gene and the second recombination site is inserted in between the sequences of a fifth viral gene and a sixth viral gene.

4. The vector system according to the preceding claims, wherein at least one of the at least two recombination sites is inserted in between the sequences of two tail-to-tail oriented genes and/or wherein the DNA with sequences of a bacterial plasmid, and preferably the non-viral part is inserted in between the sequences of two tail-to-tail oriented genes.

5. The vector system according to the preceding claims, wherein the DNA with sequences of a bacterial plasmid is inserted in between the sequences of the UL3 gene and the UL4 gene and/or wherein one of the at least two recombination sites is inserted in between the sequences of the UL10 gene and the UL11 gene, wherein preferably the second recombination site is inserted in between the sequences of the UL55 gene and the UL56 gene.

6. The vector system according to the preceding claims, wherein the non-viral part comprises DNA-sequences of the plasmid pBeloBAC11.

7. The vector system according to the preceding claims, wherein the DNA with sequences of a bacterial plasmid can be removed after transfection in mammalian cells by recombination.

8. Use of the vector system according to the preceding claims for the generation and/or production of a transgenic virus.

9. Method for the production of a transgenic virus comprising the steps:
a) introducing a transgene expression cassette into one of the at least two recombination sites of the vector system according to claims 1 to 7 to obtain a vector encoding a transgenic virus;
b) transfecting the vector obtained in step a) into a mammalian cell;
c) cultivating the transfected mammalian cell;
d) isolating the virus produced in the mammalian cell.

10. Method according to claim 9, wherein in step a) a first transgene expression cassette is introduced into the first of the at least two recombination sites and a second transgene expression cassette is introduced into the second of the at least two recombination sites of the vector system according to claims 1 to 7.

11. Method according to claim 9 or according to claim 10, wherein step a) is performed in a prokaryotic system.

12. Method according to claims 9 to 11, wherein the transgene cassette encodes at least one protein or at least one peptide or an RNA, preferably a protein or a peptide selected from the group consisting of target control proteins, immunomodulating agents like immune-checkpoint inhibitors or cytokines, anti-cancer peptides (ACP) or proteins, pro-apoptotic proteins, extracellular matrix degrading proteins, proteins for the tumor-antigene-presentation, pathogen antigens and combinations thereof.

13. Method according to claims 9 to 12, for the production of a vaccine or an oncolytic virus or viral vector for gene therapy.

14. Virus obtained in the method according to claims 9 to 13.

15. Virus according to claim 14 for the use as vaccine or as oncolytic virus or in a gene therapy.
